# EUROPEAN PATENT SPECIFICATION

(11) **EP 2 185 004 B1**
(45) Date of publication and mention of the grant of the patent: **15.03.2017**
(21) Application number: 08793894.0
(22) Date of filing: 04.08.2008
(51) Int. Cl.: A23J 1/04

(54) **AMINO ACID AND PEPTIDE PRODUCTS**
AMINOSÄURE- UND PEPTIDPRODUKTE
PRODUITS À BASE D'ACIDES AMINÉS ET DE PEPTIDES

(30) Priority: 03.08.2007 US 953975 P
(43) Date of publication of application: 19.05.2010
(73) Proprietor: Aminotech AS, 2665 Lesja (NO)
(72) Inventor: ERMOLIN, Gennady, A., S-435 32 Mölnlycke (SE); GLASMÄSTER, Karin, S-422 41 Hisings Backa (SE); NESSE, Knut, O., Westmount Quebec H3Y 2M4 (CA); RONG, Chunjun, NO-5009 Bergen (NO)
(74) Representative: Livgard, Kim Are Birkeli
(86) International application number: PCT/NO2008/000282
(87) International publication number: WO 2009/020394

(56) References cited:
- WO-A1-2004/021797
- AU-B2- 605 797
- FR-A- 2 168 259
- GB-A- 2 136 002
- US-A1- 2004 038 391
- US-A1- 2004 248 239
- WU HUI-CHUN, CHEN HUA-MING, SHIAU CHYUAN-YUAN: "Free amino acids and peptides as related to antioxydant properties in protein hydrolysates of mackerel (Scomber austriasicus)" FOOD RESEARCH INTERNATIONAL, vol. 36, no. 9-10, 1 January 2003 (2003-01-01), pages 949-957, XP002502818 Oxford (UK)

## Description

### TECHNICAL FIELD

The present invention relates to compositions comprising amino acids and short peptide products and methods for forming same.

### BACKGROUND AND DEFINITIONS

Amino acids and peptides are well known within the pharmaceutical, natural medicine and veterinary medical industry as components of products like parenteral nutrition as well as special nutrition to treat certain trauma. Up to now the main products used have been extracts from blood plasma and protein hydrolysates produced by means of pancreatic enzymes from pigs and calves. The present invention provides the pharmaceutical and food industry with a new product comprising a unique combination of amino acids and short peptides.

Amino acids and ultra short peptides are also used for biotechnological processes, for example, when a highly potent culture medium is produced. A limitation for cultivating single cell organisms or cell substrates from higher organisms is the access to culture media of adequate quality. Lack of suitable media and high price are also limiting factors. Moreover, amino acids or peptides produced by biotechnological methods generally contain growth-inhibiting substances. These components are not significantly present in the products of the invention. The combination of natural amino acid profiles and biological trace elements and minerals in the product according to the present invention is a unique product for the preparation of culture media for the biotechnological industry.

Peptides/amino acids are used in the food processing industry as binders, emulsifiers, and taste enhancers or similar. The applications are considerable and growing. Most of the peptides and amino acids used in the industry come from soya beans and milk. Amino acids and peptides from soya and milk in particular are known for causing allergenic reactions, which can only be avoided by using another peptide/amino acid composition which does not originate from these sources, or a peptide/amino acid composition from soya or milk that has been sufficiently altered in order not to cause such allergic reactions. There is a strong demand for a composition of amino acids and peptides which does not cause allergenic reactions. Products from most animal sources have not attained the same degree of utilisation as there are no extraction techniques that can preserve the functionality of the product and at the same time remove the unwanted, quality diminishing components like salt and fat.

Within feed production there exists a number of different combinations of proteins, peptides and amino acids originating from a variety of sources. The composition of peptides, amino acids and proteins within feed products is also very important since the growth of the animals is dependent on a balanced feed intake. Thus, in this area, there is also a strong demand for a composition that provides optimal growth conditions for the animals.
GB 2136002 describes a process in which Rawfish and/or shellfish is extracted to yield products having pharmaceutical and/or nutritional functions. Fish is heated to remove smell, and treated in sequence with proteases
WO 04/021797 describes a method for recovering peptides or amino acids, oils or fats from raw animal or aquatic materials.
US 2004/038391 describes a method of production in which amino acids and very short peptides are produced from cold water animals leading to enzymatic protein hydrolysate.
Hui-Chun et al., (Food Res Int., Vol. 36, no. 9-10, p.949-957; 2003) describe free amino acids and peptides in protein hydrolysates of mackerel.

In the following the term "endogenous" enzymes is used as a term for the enzymes originating within the protein product as opposed to the "exogenous" enzymes which are extraneous enzymes added to the raw protein material during traditional hydrolysis. One example of an "exogenous" enzyme is "Deterzyme APY", which is a bacterial protease (E.C. 3.4.21) prepared by controlled fermentation of *Bacillus alcalophilus,* and which can be purchased from a number of suppliers.

The term "endogenous" enzymes are also used to mean enzymes extracted from other similar natural enzyme materials or raw materials, preferably from cold-blooded animals.

In the following the term "free" in connection with amino acids is used to describe single amino acids not bound to other amino acids.

The term "short" with respect to peptides is used here to define peptides with a molecular size of less than 10000 Dalton, prefereably less than 5000 Da.

The term "fat" in this context is meant to include any type of oil or fat, originally included in the protein source.

The term "full range" in connection with amino acids is used here to define a mixture of natural amino acids covering all 20 amino acids necessary for human protein synthesis, including alanine, arginine, asparagine, aspartic acid, cysteine, glutamic acid, glutamine, glycine, histidine, isoleucine, leucine, lysine, methionine, phenylalanine, praline, serine, threonine, tryptophan, tyrosine and valine; and 6 amino acids, including cystine, hydroxylysine hydroxyproline, ornithine, taurine and thyroxine, which are vital to other human metabolisms.

The term "natural amino acids" is used to describe the naturally occurring L isomeric form.

The term "pharmaceutical quality" is used to describe products for intravenous use and products that are classified as medicine for humans and animals or natural medicine. The term "biotechnological quality" is used to describe products that can be used, for example, as culture media or catalysts in the culturing of cells, bacteria, fungi and algae.

The term "foodstuff quality" is used to describe products that are used for human consumption either as an additive or as an independent product.

### DESCRIPTION OF VARIOUS EMBODIMENTS

The amino acid and short peptide products according to the present invention as defined in claim 1 are illustrated by a description of a product here referred to as Amizate, but this invention is not limited to this product as such.

A product as described further herein is a full-range, protein-free, amino acid - peptide product made from fish raw material, which is a safe and rich source of protein, an example of which is referred to as Amizate.

In one embodiment, the product may be produced by processes described in Norwegian patent no. 317900, US patent application no.10/523,151 and/or US application no. 11/678,543, and these documents are fully incorporated herein by reference. In such processes the fish protein is pre-digested to amino acids and peptides in a natural way very similar to human digestion (utilizing serine proteases, enzymes found in the fish itself). This process of inducing and maintaining digestive action by natural enzymes is an important scientific milestone, never before achieved in commercial scale.

The result of the processes is a natural and wholesome blend of free amino acids and short peptides that fit into the human digestive cycle, bringing profound effects for health and well-being.

A product such as Amizate extracted from fish protein delivers the full range of amino acids and essential short peptides needed for human protein synthesis and metabolism. A product such as Amizate contains all non-essential amino acids (which can normally be synthesized in the body), and all essential amino acids (provided only in the diet) for optimal health. But it takes a healthy body to synthesize the non-essential amino acids. The profile of a product such as Amizate meets the physiological needs of the human body. Possible amino acid/peptide shortages and imbalances are thereby naturally corrected.

A product such as Amizate does not contain potentially harmful substances often found in amino acid products from milk (lactose, to which many people have intolerance), soy (isoflavones), meat (substances which can cause BSE) or other raw materials.

These process of manufacturing amino acids is uniquely natural, maximizing the nutrient content and broad amino acid range of the original fish protein.

The natural mixture of enzymes used for the protein digestion ensures an almost complete hydrolysis. The result is a product with essentially only free amino acids and short peptides which can directly be used in the building tasks of the body.

In the prior art, typically amino acid products are produced using synthetically modified enzymes, which are barely able to hydrolyze 20% of the protein. Such amino acid products neither contain the full range nor the balanced content of a product such as Amizate. A more complete hydrolysis of such products gives a bitter taste and odor not present in a product such as Amizate.

The product according to the present invention in a different embodiment has a total amount of amino acids as follows:
40-70 g Alanine, 45-60 g Alanine, or 45-57 g Alanine per kg product;
25-60 g Arginine, 30-45 g Arginine, or 31-44 g Arginine per kg product;
40-85 g Asparagine + Aspartic Acid, 40-70 g Asparagine + Aspartic Acid, or 45-68 g Asparagine + Aspartic Acid per kg product;
0-10 g Cystine, 1-7 g Cystine, or 1.5-7 g Cystine per kg product;
80-115 g Glutamic Acid + Glutamine, 80-105 g Glutamic Acid + Glutamine, or
85-100 g Glutamic Acid + Glutamine per kg product;
40-70 g Glycine or 45-68 g Glycine per kg product;
8-30 g Histidine, 8-25 g Histidine, or 10-20 g Histidine per kg product;
15-45 g Isoleucine, 18-40 g Isoleucine, or 20-39 g Isoleucine per kg product;
35-70 g Leucine, 35-65 g Leucine, or 40-64 g Leucine per kg product;
35-65 g Lysine, 40-60 g Lysine, or 42-58 g Lysine per kg product;
10-30 g Methionine, 12-25 g Methionine, or 15-22 g Methionine per kg product;
15-45 g Phenylalanine, 18-40 g Phenylalanine, or 20-38 g Phenylalanine per kg product;
15-50 g Proline, 25-45 g Proline, or 29-40 g Proline per kg product;
15-40 g Serine, 18-40 g Serine, or 20-36 g Serine per kg product;
15-45 g Threonine, 18-40 g Threonine, or 22-38 g threonine per kg product;
1-15 g Tryptophan, 5-15 g Tryptophan, or 5-10 g Tryptophan per kg product;
10-40 g Tyrosine, 15-35 g Tyrosine, or 16-30 g Tyrosine per kg product;
20-55 g Valine, 20-50 g Valine, or 26-49 g Valine per kg product.

Amizate's fully realized natural hydrolysis ensures high bioavailability, i.e., a very rapid and complete absorption by the body.
The body requires a full range of amino acids in sufficient amounts in order to make proteins and provide for other physiological needs.

Amino acids available for new protein formation are either "free" (stand alone) or in short peptide chains in combination with other amino acids. Both are required and are indispensable for different metabolic processes and both are present in balanced measure in a product such as Amizate.

In one embodiment, used as a supplement, a product such as Amizate provides adequate amounts of all amino acids, thereby eliminating the need for special dietary planning.

Amizate is a powerful restorative force for the human body and can as an example contribute to general health & well-being in the following ways:
- Improvement of Immune Function
- Anti-Oxidant Protection
- Fat Metabolism
- Muscle Mass
- Endurance

These effects are based on at least the following knowledge: During periods of stress, illness or intense physical activity, the body does not make enough non-essential amino acids to meet its demands. It then depends more heavily on dietary sources.

The following are some promising areas of amino acid research for treatment of:
1) Cerebral & Nervous System Disorders;
2) Wound Care, Skin Care, Skin Diseases (e.g., Psoriasis);
3) Sleep Disorders (e.g., using L-Tryptophan);
4) Depression (e.g., using L-Phenylalanine, L-Tyrosine); and
5) Alcoholism (e.g., using L-Glutamic Acid, L-Glutamine)

Amino acids and short peptides are of importance far beyond just building proteins. For example, amino acids act as precursors to neurotransmitters, the chemicals which carry messages from one nerve to another or to a target tissue. They also combine with other molecules to form coenzymes.
Specific amino acids stimulate production of chemicals or initiate actions that protect, cleanse or otherwise benefit the body such as inhibiting tumor development or growth, detoxifying ingested poisons, clearing bronchial mucus, protecting against toxic effects of radiation, copper, smog and tobacco smoke, reducing alcoholic cravings and generally supporting growth and repair mechanisms throughout life.

The products according to the present invention are new combinations of free amino acids and peptides which are different from proteins, or single amino acids.

the products comprise all 20 amino acids necessary for human protein synthesis, including all 8 indispensable amino acids (Isoleucine*, Leucine*, Lysine, Methionine, Phenylalanine, Threonine, Tryptophan, Valine*), and the 2 semi-indispensable amino acids Arginine and Histidine (indispensable for children). (The particularly valuable branched chain amino acids are marked with *).

In another embodiment the products further comprise Cysteine (indispensable for premature babies).
In yet another embodiment the product further comprises 6 additional amino acids vital to other human metabolisms: Cystine, Hydroxyleucine, Hydroxyproline, Ornithine, Taurine, and Thyroxine.

In yet another embodiment, a product such as Amizate provides over 60% of amino acids in free form and the remainder in short peptides (< 10 000 Dalton). No amino acids remain bound in proteins or large molecules requiring digestion.

In another embodiment, a product such as Amizate is a mild-tasting, off-white powder easily incorporated into virtually any food or beverage, or is ingested directly in capsule form.

In another embodiment, the product according to the present invention may be included in customized foods, beverages and yogurts targeted at, for example, geriatrics, infants, or athletes and fitness enthusiasts.

A product such as Amizate is a food product, and has regulatory approval as such from the Norwegian Food Safety Authority.

In Table 1 Amizate, an embodiment of the products according to the present invention is compared with known amino acid and peptide sources.

**Table 1: Competitive characteristics of Amizate vs other hydrolyzed proteins and bacteria-produced amino acids in use for building dietary proteins**

| | Amizate | Hydrolyzed Fish | Whey | Soy | Bacteria |
|---|---|---|---|---|---|
| Full functionality in physiology | Yes | No | No | No | No |
| Absorption to blood | 100% | < 60% | about 50% | about 40% | 0 - 100% |
| Amino Acids present in free form | 26 | 26 | 18 | 14 | about 16 |
| Calibration essential and none essential amino acids | Optimal | Good | Not optimal | Not optimal | Risk calculation |
| Short peptides present | All | Some | Very few | Very few | None |
| Endogenous enzymes in the process | Yes | No | No | No | No |
| No whole proteins left in product (contamination risk) | Yes | No | No | No | Yes |
| No long peptides left in product | Yes | No | No | No | Yes |
| No allergic reactions | Yes | No | No | No | Probable |
| Acceptable taste | Yes | No | No | No | No (?) |
| No masking required by industry (bad taste) | Yes | No | No | No | No |
| Ideal for transport of nitrogen (nitrogen balance) | High | Medium | Medium | Medium | Unknown |
| Ideal for compensation against Limiting Amino Acid | High | Medium | Medium | Medium | High/none |
| Production of Structural proteins | Ideal | Some | Few | Few | Few |
| Production of Contractile proteins | Ideal | Some | Few | Few | Few |
| Production of Specialized proteins: | | | | | |
| • Enzymes | Ideal | Limited | Limited | Limited | Limited |
| • Hormones | Ideal | Limited | Limited | Limited | Limited |
| • Antibodies | Ideal | Limited | Limited | Limited | Limited |
| Amino acids as precursors to neurotransmitters | Ideal | Limited | Limited | Limited | Partial only |
| Balance of branched Chain Amino Acids | Ideal | Limited | Limited | Limited | Partial only |
| Recommended for physiological use | Life long | Limited time | Limited time | Limited time | Limited time |
| Consumption of energy by user for use of product | Ultra low | Medium | Medium | Medium/high | Low/high |

### Enzymatic protein hydrolysate from fish

Today the present applicant has succeeded in developing new technology (autolysis) for making hydrolysate. This patented technology uses serine protease (enzymes) from fish intestines to digest fish proteins. The source of enzymes for Aminotech is "unlimited". This makes it possible to make large-scale hydrolysate production. The protein source is meat of fish, which contains between 13 - 24 % crude proteins. The amino acid structure from fish is close to the structure in animal meat (poultry and egg protein)

Fish proteins are excellent and even preferred as raw material for the manufacture of an optimally balanced hydrolysate, which can find wide applications within the food-processing industry, pharmaceutical industry, biotechnology and in the agricultural sector. In addition, the hydrolysates are of great interest for many research institutions which use amino acid sources in their R&D.

**Amino acid structure of proteins from selected fish, poultry and hen's eggs. (mg per 100g of raw material)**

| **Amino acids** | **Fish** | | **Poultry** | | **Hen's eggs (whole)** |
|---|---|---|---|---|---|
| | *Capelin (Lodde)* | *Herring* | *Chicken* | *Goose* * *(category II)* | |
| **Essential amino acids:** | **5360** | **7500** | **7310** | **6641** | **5243** |
| Valine | 900 | 1000 | 946 | 913 | 772 |
| Isoleucine | 570 | 900 | 760 | 775 | 591 |
| Leucine | 1300 | 1600 | 1483 | 1445 | 1081 |
| Lysine | 1090 | 1800 | 1700 | 1436 | 903 |
| Methoinine | 410 | 350 | 510 | 413 | 424 |
| Theronine | 610 | 900 | 849 | 726 | 610 |
| Tryptophan | 160 | 250 | 315 | 212 | 204 |
| Phenylalanine | 560 | 700 | 747 | 721 | 652 |
| **Nonessential amino acid:** | **6960** | **11800** | **12210** | **19461** | **7362** |
| Alanine | 790 | 1200 | 1239 | 1100 | 710 |
| Arginine | 830 | 1200 | 1275 | 1151 | 787 |
| Aspartic acid | 1200 | 2000 | 1832 | 1460 | 1229 |
| Histidine | 330 | 500 | 573 | 350 | 340 |
| Glycine | 710 | 1100 | 1348 | 1144 | 416 |
| Glutamic acid | 1360 | 3000 | 3117 | 2720 | 1773 |
| Hydroxyprolin | traces | traces | 171 | 356 | 14 |
| Proline | 480 | 700 | 959 | 787 | 396 |
| Serine | 570 | 1000 | 859 | 672 | 928 |
| Tyrosine | 500 | 800 | 630 | 582 | 476 |
| Cystine | 170 | 300 | 207 | 139 | 293 |
| **Total quantity**, **g%** | 12,32 | 19,30 | 19,52 | 17,10 | 12,60 |
| **Protein, %** | 13,1 | 19,1 | 19,7 | 17,0 | 12,7 |

| | | | | | |
|---|---|---|---|---|---|
| This table shows that amino acids from fish compare very favourably with other sources, including control proteins from eggs. * Category II refers to geese with minimum fat content | | | | | |

### Brief characteristic of Amizate

Until now intestines from fish as a source proteolytic enzymes has not been used. Intestines and other by-products from fish are today routinely used in the fishing industry for making fish oil and fish flour.

Aminotech's production process involves grinding fish raw materials, including proteolytic enzyme sources (fish intestines). After grinding, the mass is diluted with water and then transported into temperature-controlled bio-reactors where an enzymatic digestion of all proteins in the source materials take place under continuous mixing and precise control conditions until amino acids and very short peptides are produced. The resulting hydrolysate is sterilised, then fish-oil and solid particles removed by centrifuge. The product is thereafter filtered, concentrated and dried to powder.

The dry product (powder) Amizate consists of 70 % amino acids (more than 50% in free form), short peptides, vitamins, minerals and microelements. This is a very high biological value product because in addition to the complete set of amino acids (26 amino acids, characteristic for tissues for humans and animals), it also has an ideal or optimal relationship between the irreplaceable (essential) and replaceable (non essential) amino acids. In addition, Amizate also contains the whole complex of vitamins group B as well as the whole set of minerals and microelements that are necessary for the physiology of the body.

Free form amino acids are those that have not chained together to form peptides or proteins; they are singular entities. Short peptides can have as few as 2 or up to 12 or more amino acids chained together. Free form amino acids and short peptides are immediately absorbed through the epithelial cells of the duodenum, and through the intestinal walls.

The pool of biologically active short peptides in a product such as Amizate contains its activator components (ability to penetrate to blood). The blood transports the activator components to different organs and tissues where they connect to so called "receptors" on the cells' surface and then switch on different salutary influence on metabolic pathways. The same activator components also influence on the cardio-blood vessels as well as the immune, endocrine and nervous systems of organisms.

Fish-oil and solid particles are separate products.

Aminotech's production process is waste-free and does not pollute the environment.

Standard bulk analysis of enzymatic protein hydrolysate at large-scale manufacture using the above-described technology gives results, for example, as follows:
Total Nitrogen (Kjeldahl) 5.0 % - 15.0 %
Amino Nitrogen (formol titration) 1 % - 10 %
Dried matter 90-99 %
Ash 2 % - 20 %
Crude Protein (Nt x 6.25) 30 % - 90 %
Dry hydrolysate contains 30 - 90 % amino acids of which 40 - 95 % of the amino acids are in free form (free amino acid).

### Possible uses of Amizate

An enzymatic protein hydrolysate such as Amizate can be used in an endless number of ways. Below is shown a number of examples.
✔ Everyday nutrient additive for people that fail to receive a complete set of animal proteins in usual food.
✔ Balanced component of baby nutrition diet.
✔ Additive in various kinds of food products, intended mainly for children and teenagers, which will compensate for deficits of essential amino acids, vitamins group B and vital minerals.
✔ Component in special food for elderly people. Elderly people are often not able to benefit sufficiently from standard food. Hydrolysate additive will give long time improvement in quality of life.
✔ Balanced source of amino acids and biologically active peptides for sportsmen from amateurs up to professionals will fuel the muscle growth process at the fastest rate possible.
✔ Balanced source of amino acids and biologically active peptides for people regularly occupied with manual labor will enable them to maintain a high level protein and also a high level of energy.
✔ General food additive to correct insufficient nutritional values. This addresses a universal problem today. By adding small amounts of product in the usual food, the nutritional values can be restored. Will for example serve as a prophylactic against essential amino acids deficits.
✔ Base component in special diet against obesity. This can be used both in a prophylactic and therapeutic way.
✔ Very compact high-energy food is especially necessary for people exposed to extreme physical conditions - for example: astronauts, mountain-climbers, tourists, seamen and military units. Dried hydrolysate contains all necessary building material and energy components needed to maintain a high quality. The level of milk acids in muscles is quickly reduced with intake of product. A small volume of product will enable the user to work "indefinitely" without reducing capacity.
✔ Ideal "emergency ration" in case of natural calamity, accidents and other disasters. It can be used as a national strategic reserve food supply.
✔ "Super-fast" food for all persons suffering from protein starvation in zones of ecological disasters, mass migrations of people and wars.
✔ Base component in enteral and parenteral nutrition of patients with different types ofserous diseases.
✔ Basic component of biologically active food additive for effective stop of abstinence syndrome.
✔ Highly effective food additive in complex treatment of a number mental and neurology diseases
✔ Medicine to cure protein insufficiency - for example: hypoproteinism, emaciation
✔ Medicine to cure protein insufficiency - for example: stomach-intestine diseases with infringement absorption of amino acids, intestinal obstruction, intoxication, burn disease, languidly granulating wounds, radial illness etc.
✔ Basic ingredients in diets for patients suffering from various syndromes - for example: short bowel syndrome, Crohn's disease, malabsorption syndromes, hepatic and pancreatic insufficiency, wasting diseases or severe trauma following surgery, burns etc.
✔ Special diets for patients suffering from metabolic disorder
✔ Basic component of diet for infants with allergy to milk protein and lactose;
✔ Basic component of diet for the patients suffering by parasitosises of intestine, liver, and pancreas (for example, at helminthes invasions: clonorhiasis and opisthorchiasis (Large problem in for example: Russia, China and Vietnamam)
✔ Highly effective biogenic medical preparation: ointments, creams, jellies, and sprays. Improves metabolic processes and acceleration of regeneration of tissue of for example: trophic ulcer, gangrene, bedsores, burns, radiating ulcers, skin transplantant, and also at various forms of dermatitis.
✔ Component in cosmetic creams, containing enzymatic protein hydrolysate, which ensures local (external) nutrition for skin's cells. Free amino acids, biologically active peptides, vitamins, minerals and the microelements of hydrolysate penetrate into cells of skin and intercellular space. Such creams stimulate normal reproduction of skin cells and increase the life of skin cells.
✔ Basic component in hair gel, shampoos etc. Enzymatic protein hydrolysate protects skin and hair against adverse internal and external factors. Free amino acids and bioactive peptides fundamentally promote growth and quality of hair.
✔ Indispensable base component of culture media for microbiology, fermentation and biotechnology.

**Competitive Physiology Characteristics of different types of Hydrolysates and Synthetic imitations**

| | ***Enzymatic Hydrolysis Biochemical procedure*** | | | ***Acid Hydrolysis*** | ***alkaline hydrolysis*** | ***Synthetic Amino Acids mix*** |
|---|---|---|---|---|---|---|
| **Components** | **Alkaline serine protease from fish** | **Acid Protease from animals** | **Alkaline and neutral protease from Bacteria** | **Chemical procedure** | **Chemical procedure** | **Chemical products** |
| | **(Aminotech AS)** | | | | | |
| ***Positive composition*** | | | | | | |
| Free amino acids (AA) | All | Limited | All | Limited | Limited | All |
| Essential amino acids | All | Limited | All | Limited | Limited | All |
| Non Essential amino acids | All | Limited | All | Limited | Limited | All |
| Calibration essential - non essential AA | Physiology optimal | Physiology not optimal | Physiology not optimal | Absent | Absent | Constructed profile |
| Short peptides (Effectors) | Very good | Limited | Not optimal | Absent | Absent | Absent |
| Vitamin - Group B | All present | Partially present | Partially present | Traces only | Traces only | Constructed added |
| Minerals and trace elements | All present | Partially present | Partially present | Partially present | Partially present | Constructed added |
| | | | | | | |
| ***Negative composition*** | | | | | | |
| Long peptides (allergy) | Absent | Very high | Absent | Absent | Absent | Absent |
| Antibiotic and antibiotic fragments (allergy) | Absent | Absent | Present | Absent | Absent | Absent |
| Deficits of physiology short peptides | None | Yes | Yes | Yes | Yes | Yes |
| Recommended physiology use | All life | Limited time | Limited time | Limited time | Limited time | Limited time |
| | | | | | | |
| ***Overall recommendation*** | | | | | | |
| Physiology use | ***** | ** | ** | * | * | ** |

### Multifunctional biologic role of peptides.

Proteins are synthesized as a result of formation of the secondary amide links between carboxyl groups and amino groups of the neighboring amino acids. Such connections refer to as peptide bonds, and the structures resulting formation of peptide bonds between residues of amino acids, are peptides. A peptide of two amino acids is named a dipeptide; a peptide of three amino acids is called tripeptide, etc.

From hydrolysis of proteins, a quantity of heterogeneous peptides are formed, and free amino acids are produced as well. The hydrolysate is a mixture consisting of free amino acids and peptides. Also in this mixture are simple saccharum and polysaccharides, fragments of nucleic acids, vitamins, minerals and microelements.
When hydrolysates are utilized in the capacity of nutrition, food supplements, feed additives and substrates for cultivation of cells and microorganisms, peptides represent supplies of amino acids, just as free amino acids are supplies of nitrogen, carbon and sulfur.

Peptides as a source of amino acids are more effective than synthetic mixtures of amino acids. F.J. Sussman and C. Gilvard (1971) have established that bacteria consume polypeptides more actively than free amino acids. They explained the phenomena of self-contained transport for polypeptides. Pittman K.A. et al. (1967) showed that Bacteroides ruminicola do not consume free proline from medium, but utilize proline-inclusive peptides.

Analogously, scientists observed a faster absorption, in the small intestines, of peptides in comparison with free amino acids (Grampton R.F., 1970), and of products of enzymatic hydrolysis in comparison with products of acid hydrolysis of the same proteins or with mixes of free amino acids (Silk D.B.A. et al. , 1973). Amino acids pass through cellular barriers against a concentration gradient that testifies to presence of the mechanism of active transport in an intestines. Thus many authors observed the phenomena of a competition for these transport resources. So, transport of the basic amino acids is activated in the presence of neutral amino acids, but absorption of neutral amino acids is inhibited in the presence of the basic amino acids (such as lysine).

As H.Newey and D. Smith (1962) have shown and as it has been confirmed in subsequent years, peptides and amino acids are transported in an epithelium of an intestine with the help of different mechanisms, in different plots of a thin intestine. Transport of low molecular weight peptides descends at participation of the hydrolases, which are taking place in structure of enterocytes.

Thus, for a higher organism als, and for microorganisms, that there are specific mechanisms of mastering and transmission of peptides. Humans and animals have stray patches of an intestine where peptides are absorbed; microorganisms have permease for transit through cellular walls. As a result of it, peptides are not connected with separate attitudes of antagonism of amino acids and are more easily assimilated.

Now the big attention is allocated to studying a role of peptides in functioning living organisms. The big quantity of the natural biologically active peptides produced by an organism in norm and at various pathologies is investigated. Biologically active peptides contain in various organs and tissues, are formed at natural biological processes in blood and a gastrointestinal tract. It is known, that peptides carry out intermediary roles between different basic regulation systems of an organism of the highest animals: nervous, humoral and immune. They are material bearers of information and they themselves exert regulation effect. Hundreds peptides are fixed, each of which is characterized by polyfunctionality of act (Ashmarin I.P., 1985). From number regulation peptides it is possible to secure {discharge} some functional groups. These are the peptide hormones produced by organs of endocrine system; tissue hormones are called quinines; numerous group of peptides as immunomodulating factors are called immunohormons and also immunomodulating factors and mediators of an exogenous origin (Ershov F.I., Malinovskaja V.V., 1996). Hormones are secreted from endocrine glands, such as thyroid and parathyroid glands; pancreas, suprarenal glands and sexual gonads, and " serve" organs which produce them; hormones of a hypophysis make control of activity of other hormones. The structure of the majority of them for a long time is investigated. They represent linear or branched peptides, in small size. So, oligopeptide hormones of a hypophysis have M.W. 1500 - 6000 D and consist of 13 - 39 amino acids.

The biologically active peptides which are formed during digestion of various proteins share in the mechanism of satiety, influence on gastro-intestinal tract, stimulate immune system, carry out other functions, for what have received the name of "alimentary hormones" (Morley J.E., 1982).

### Amino Acid Requirements

Adult requirements for indispensable amino acids have been estimated with the nitrogen balance procedure in the same way one establishes protein requirements, except that proteins in the basic diet are replaced by a mixture of amino acids so the quantity of each amino acid can be adjusted separately. Diets containing adequate quantities of all by one indispensable amino acid and increasing increments of the missing one are then fed in sequence to experimental subjects, and the intake at which zero nitrogen balance is achieved, as in estimating the protein requirement, is taken as the requirement for the indispensable amino acid. This process was used to determine the adult requirements for ten of the indispensable amino acids.

The indispensable amino acid requirements of infants and young children have been estimated by observing changes in weight after the amount of one amino acid in an otherwise fully adequate amino acid diet is reduced incrementally. If intake of one amino acid falls below the requirements, weight gain will decline. To ensure that growth will not be impaired by this procedure, the amount of the amino acid that is limiting growth is increased in the diet as soon as the first evidence of reduced weight gain is detected. Indispensable amino acid requirements of infants have also been estimated from amino acid intakes of infants growing satisfactorily, calculated from knowledge of the amounts of breast milk or formula they have consumed and the amino acid composition of these foods. Requirements determined via this procedure, except that for tryptophan, were lower than those obtained with amino acid diets.

The most recent estimates of amino acid requirements of infants, children, and adults are shown in the table below (Table III). Requirement values for infants reported by a National Research Council committee using essentially the same information ranged from 20% lower to 20% higher than the Food and Agriculture Organization (FAO)/World Health Organization (WHO) values. The values for each age grouping were, nonetheless, in the same range, and differences between the age-groups were similar.

**Table III. FAO/WHO/UNO Amino Acid Requirements (mg/kg Body Wt/day)**

| | Infants | Children | Boys | Adults |
|---|---|---|---|---|
| | (4-6 Mo) | (2 Yr) | (10-12 Yr) | |
| Histidine | 28 | NA | NA | NA |
| Isoleucine | 70 | 31 | 30 | 10 |
| Leucine | 161 | 73 | 45 | 14 |
| Lysine | 103 | 64 | 60 | 12 |
| Methionine + Cysteine | 58 | 27 | 27 | 13 |
| Phenylalanine + Tyrosine | 125 | 69 | 27 | 14 |
| Theronine | 87 | 37 | 35 | 7 |
| Tryptophan | 17 | 12.5 | 4 | 3.5 |
| Valine | 93 | 38 | 33 | 10 |
| Total amino acids | 742 | 352 | 261 | 84 |
| Proteine | 1650 | 1200 | 1000 | 750 |
| E/T | 45 | 29 | 26 | 11.2 |

| | | | | |
|---|---|---|---|---|
| NA = not available. | | | | |

Amino acid requirements decline much more rapidly with increasing age than protein requirements. Scientists, in a carefully controlled study of phenylketonuric infants, observed that, during the first 2 yr of life, the phenylalanine requirement fell by 75%, whereas the protein requirement declined by only ∼50% over this period. For most amino acids, the difference between the amino acid and protein requirements becomes greater as maturity is approached. Thus, the proportion of protein or total amino acids required as indispensable amino acids is much lower for the adult than for the infant (Table III). In other words, a protein that may not meet the indispensable amino acid requirements of the child when it is consumed in an amount that meets the total nitrogen requirement may provide amounts of amino acids in excess of the requirements for adults consuming enough protein to meet the nitrogen requirement.

Requirements of adults for several indispensable amino acids have been reinvestigated with an isotopic procedure. Adult human subjects were fed diets containing a mixture of amino acids instead of protein, with one carbon-labelled indispensable amino acid being included at a time in graded amounts in a series of diets. Expired air was collected for several hours after feeding each diet, and the amount of labelled amino acid oxidized was estimated from the amount of isotopic carbon in the expired carbon dioxide. From the values obtained for the amounts of several amino acids oxidized, the investigators concluded that adult requirements for indispensable amino acids had been underestimated by 50-75% by the nitrogen balance procedure. Other investigators questioned this conclusion, largely on the basis of theoretical considerations, but did acknowledge that amino acid requirements of adults are probably underestimated by the nitrogen balance procedure.

Largely in response to criticisms of the nitrogen balance procedure, adult protein requirements have been extensively reinvestigated and reassessed, leading to the conclusion that they had earlier been underestimated by ∼20%. It should not be surprising, therefore, if adult amino acid requirements have also been underestimated. The question is by how much. - Although the results of oxidation studies indicate that with the exception of the methionine requirement, amino acid requirements of adult humans have been underestimated by a factor of 2-3, values for amino acid requirements of young rats and piglets estimated by the oxidation procedure and growth assays have been in good agreement. Also, young adults have been maintained in nitrogen balance and good health for 50 days while consuming cereal grain diets that provided lysine at ∼50% above the current requirement. These observations raise several questions: Is the nitrogen balance procedure so much less reliable for estimating adult requirements for amino acids than for protein? If it is - why? Why are methionine requirements of adults estimated by the nitrogen balance and oxidation procedures similar but values for most other amino acid requirements so widely divergent? Is the oxidation procedure for estimating amino acid requirements for maintenance subject to sources of error that are not obvious? Unfortunately, both the nitrogen balance and oxidation procedures for estimating amino acid requirements for maintenance are indirect methods, and there is no direct method of validating them in human subjects. Thus, the question of amino acid requirements for adults remains unresolved.

From a practical viewpoint, even if amino acid requirements of adults have been underestimated by 50-75%, the proportion of total amino acids needed as indispensable amino acids would increase only from ∼10 to 20 or 30%. Therefore, any protein that meets the indispensable amino acid needs of young children would be more than adequate for adults, provided that the amount consumed was sufficient to meet the nitrogen requirement.

### WORKING EXAMPLES:

The following examples are include to further illustrate the present invention and are not to be construed as limiting.

### Examples 1-4:

The table below lists analytical results from four different production batches (examples) from the production facility at Lesja.

| **Analytical Results** | **Example 1** | **Example 2** | **Example 3** | **Example 4** |
|---|---|---|---|---|
| **Batch no.** | **240407A1A** | **080507A1A** | **220507A1A** | **190607A2A** |
| **Parameter** | Powder 26 April 2007 | Powder 10 May 2007 16.25-20.00 | Powder 23 May 2007 13.30-19.00 | Powder TD 21 June 2007 |

| | % | % | % | % |
|---|---|---|---|---|
| Raw ash | 14.3 | 14.5 | 13.7 | 18.8 |
| Raw fat content | 0.3 | 0.2 | 0.3 | 0.6 |
| Dry matter | 96.9 | 97.9 | 98.2 | NA |
| Amino-N | 7.3 | 7.3 | 7.2 | NA |
| Total N | 11.68 | 11.8 | 12 | NA |
| | | | | |

| **Total amino acids** | **g/kg** | **g/kg** | **g/kg** | **g/kg** |
|---|---|---|---|---|
| Cystine | 6.2 | 5.1 | 5.9 | 2.2 |
| Methionine | 19.2 | 19.6 | 19.6 | 16.7 |
| Asparagine+Aspartic Acid | 58.9 | 65.2 | 65.1 | 48.8 |
| Threonine | 33.6 | 34.1 | 35.4 | 24.5 |
| Serine | 26.3 | 30.7 | 33.4 | 22.5 |
| Glutamic Acid+Glutamine | 93.9 | 94.5 | 93.6 | 87.0 |
| Proline | 33.1 | 34.9 | 33.6 | 33.6 |
| Glycine | 49.8 | 48.3 | 52.7 | 64.6 |
| Alanine | 50.4 | 51.1 | 53.8 | 50.5 |
| Valine | 46.2 | 45.2 | 44 | 29.1 |
| Isoleucine | 34.9 | 33.9 | 35.6 | 22.1 |
| Leucine | 59.4 | 60.2 | 60.2 | 42.9 |
| Tyrosine | 26 | 27.8 | 27.6 | 19.1 |
| Phenylalanine | 30.6 | 31 | 32.1 | 22.7 |
| Histidine | 17 | 17.7 | 16.7 | 12.7 |
| Lysine | 47.4 | 52.3 | 54.9 | 50.9 |
| Arginine | 36.5 | 33.3 | 34.1 | 41.7 |
| Tryptophan | 8.4 | 8.7 | 8.9 | 5.1 |
| | | | | |

| **Free amino acids** | **g/kg** | **g/kg** | **g/kg** | **g/kg** |
|---|---|---|---|---|
| Cystine | 5.9 | NA | 1.5 | <0.1 |
| Methionine | 17.5 | 20.2 | 18.9 | 12.1 |
| Asparagine+Aspartic Acid | 24.1 | 34.7 | 26.4 | 13.1 |
| Threonine | 36.8 | 37 | 40.3 | 14.7 |
| Serine | 21.4 | 26.4 | 28.4 | 7.9 |
| Glutamic Acid+Glutamine | 55 | 52.1 | 43.2 | 32.5 |
| Proline | 11.5 | 12.4 | 7.2 | 3.6 |
| Glycine | 26.7 | 27.6 | 27.6 | 18.2 |
| Alanine | 40.9 | 46.9 | 46 | 31.1 |
| Valine | 40 | 45.2 | 40.5 | 18.9 |
| Isoleucine | 28.3 | 30.3 | 28.9 | 13.0 |
| Leucine | 50.6 | 56 | 54.7 | 28.0 |
| Tyrosine | 23.8 | 23 | 18.5 | 13.4 |
| Phenylalanine | 25.6 | 27.2 | 23.8 | 14.4 |
| Histidine | 14 | 15 | 12.3 | 11.5 |
| Lysine | 38.8 | 46.8 | 41.9 | 26.4 |
| Arginine | 30.9 | 30.6 | 27.1 | 21.4 |
| Tryptophan | NA | NA | NA | 3.5 |
| | | | | |

| **Inorganic compounds** | **mg**/**kg** | **mg/kg** | **mg**/**kg** | **mg/kg** |
|---|---|---|---|---|
| Sodium | NA | 45234 | 40100 | 50075 |
| Phosphorous | NA | 9555 | 7820 | 12644 |
| Chromium | NA | 4.6 | 0.68 | <0.1 |
| Copper | NA | 151 | 154 | 64 |
| Magnesium | NA | 336 | 231 | <20 |
| Manganese | NA | 5.3 | 2.3 | <1 |
| Selenium | NA | 10 | 9.3 | <1 |
| Zinc | NA | 1580 | 1441 | 332 |
| Calcium | NA | 126 | 105 | <20 |
| Iron | NA | 73 | 48 | 8.0 |
| Mercury | NA | <0.005 | <0.005 | <0.005 |
| Cadmium | NA | <0.05 | <0.05 | <0.05 |
| Arsenic | NA | 4.8 | 7.5 | <1 |
| Lead | NA | 0.23 | 0.1 | <0.05 |
| Potassium | NA | 15312 | 13860 | 20906 |
| Aluminum | NA | 4.1 | 3.3 | <2 |
| Chloride | NA | 2.41 | 1.84 | 3.01 |
| Sulphur | NA | 15167 | 13910 | 13910 |
| Silicon | NA | 104 | 45 | 70 |
| Vanadium | NA | 0.68 | <1 | <1 |
| Nickel | NA | 2.4 | <1 | <1 |
| Molybdenum | NA | 0.46 | 0.45 | <1 |
| Boron | NA | <10 | <5 | <10 |
| Fluoride | NA | 23 | 31 | 37 |
| | | | | |

| **Others** | **mg/kg** | | | |
|---|---|---|---|---|
| Histamine | | <0.1g/kg | 130 | <100 |

| | | | | |
|---|---|---|---|---|
| NA = not available. | | | | |

## Claims

1. A product composition being protein-free comprising a combination of amino acids and peptides, the combination consisting of free natural amino acids and short natural peptides of an aquatic animal origin, wherein
- the free amino acids comprise all 20 amino acids necessary for human protein synthesis;
- the short natural peptides have a molecular size of less than 10 kDa; and
- comprising more than 40 % by weight free amino acids, determined as the ratio of amino nitrogen by formol titration to total nitrogen by the Kjeldahl method.

2. Product according to claim 1, comprising more than 50 % by weight free amino acids.

3. Product according to claim 1, comprising more than 57 % by weight free amino acids and less than 43 % by weight short peptides, and where the amino acids and short peptides constitute more than 68 % by weight of the product.

4. Product according to claim 1, where said product comprises less than 0.7 % by weight fat, about 1-7 % by weight water and about 2-20 % by weight minerals.

5. Product according to claim 1, where said product comprises less than 0.1 % by weight fat, about 1-7 % by weight water and about 2-20 % by weight minerals.

6. Product according to any one of claims 1 to 5, where the product comprises micronutrients including trace elements and vitamins.

7. Product according to any one of claims 1 to 6, where the free amino acids comprises 26 amino acids: alanine, arginine, asparagine, aspartic acid, cysteine, glutamic acid, glutamine, glycine, histidine, isoleucine, leucine, lysine, methionine, phenylalanine, proline, serine, threonine, tryptophan, tyrosine, valine, cystine, hydroxylysine, hydroxyproline, ornithine, taurine and thyroxine.

8. Product according to any one of claims 1 to 7, wherein the short natural peptides have a molecular size of less than 5 kDa.

9. Product according to any one of claims 1 to 8, where the product is Amizate.

10. Product according to any one of claims 1 to 8, comprising a total amount of
| | |
|---|---|
| 40-70 g | Alanine per kg product; |
| 25-60 g | Arginine per kg product; |
| 40-85 g | Asparagine + Aspartic Acid per kg product; |
| 0-10 g | Cystine per kg product; |
| 80-115 g | Glutamic Acid + Glutamine per kg product; |
| 40-70 g | Glycine per kg product; |
| 8-30 g | Histidine per kg product; |
| 15-45 g | Isoleucine per kg product; |
| 35-70 g | Leucine per kg product; |
| 35-65 g | Lysine per kg product; |
| 10-30 g | Methionine per kg product; |
| 15-45 g | Phenylalanine per kg product; |
| 15-50 g | Proline per kg product; |
| 15-40 g | Serine per kg product; |
| 15-45 g | Threonine per kg product; |
| 1-15 g | Tryptophan per kg product; |
| 10-40 g | Tyrosine per kg product; and |
| 20-55 g | Valine per kg product. |

11. Product according to any one of claims 1 to 8, comprising a total amount of
| | |
|---|---|
| 45-60 g | Alanine per kg product; |
| 30-45 g | Arginine per kg product; |
| 40-70 g | Asparagine + Aspartic Acid per kg product; |
| 1-7 g | Cystine per kg product; |
| 80-105 g | Glutamic Acid + Glutamine per kg product; |
| 40-70 g | Glycine per kg product; |
| 8-25 g | Histidine per kg product; |
| 18-40 g | Isoleucine per kg product; |
| 35-65 g | Leucine per kg product; |
| 40-60 g | Lysine per kg product; |
| 12-25 g | Methionine per kg product; |
| 18-40 g | Phenylalanine per kg product; |
| 25-45 g | Proline per kg product; |
| 18-40 g | Serine per kg product; |
| 18-40 g | Threonine per kg product; |
| 2-15 g | Tryptophan per kg product; |
| 15-35 g | Tyrosine per kg product; and |
| 20-50 g | Valine per kg product. |

12. Product according to any one of claims 1 to 8, comprising a total amount of
| | |
|---|---|
| 45-57 g | Alanine per kg product; |
| 31-44 g | Arginine per kg product; |
| 45-68 g | Asparagine + Aspartic Acid per kg product; |
| 1.5-7 g | Cystine per kg product; |
| 85-100 g | Glutamic Acid + Glutamine per kg product; |
| 45-68 g | Glycine per kg product; |
| 10-20 g | Histidine per kg product; |
| 20-39 g | Isoleucine per kg product; |
| 40-64 g | Leucine per kg product; |
| 42-58 g | Lysine per kg product; |
| 15-22 g | Methionine per kg product; |
| 20-38 g | Phenylalanine per kg product; |
| 29-40 g | Proline per kg product; |
| 20-36 g | Serine per kg product; |
| 22-38 g | Threonine per kg product; |
| 4-10 g | Tryptophan per kg product; |
| 16-30 g | Tyrosine per kg product; and |
| 26-49 g | Valine per kg product. |

## Patentansprüche

1. Proteinfreie Produktzusammensetzung, umfassend eine Kombination aus Aminosäuren und Peptiden, wobei die Kombination aus freien natürlichen Aminosäuren und kurzen natürlichen Peptiden wassertierischen Ursprungs bestehen, wobei
- die freien Aminosäuren alle 20 Aminosäuren umfassen, die für menschliche Proteinsynthese notwendig sind;
- die kurzen natürlichen Peptide eine Molekülgröße von weniger als 10 kDA aufweisen; und
- umfassend mehr als 40 Gew.-% freie Aminosäuren, bestimmt durch das Kjeldahl-Verfahren als das Verhältnis von Aminostickstoff durch Formoltitration zu Gesamtstickstoff.

2. Produkt nach Anspruch 1, umfassend mehr als 50 Gew.-% freie Aminosäuren.

3. Produkt nach Anspruch 1, umfassend mehr als 57 Gew.-% freie Aminosäuren und weniger als 43 Gew.-% kurze Peptide, und wobei die Aminosäuren und kurzen Peptide mehr als 68 Gew.-% des Produkts ausmachen.

4. Produkt nach Anspruch 1, wobei das Produkt weniger als 0,7 Gew.-% Fett, etwa 1 bis 7 Gew.-% Wasser und etwa 2 bis 20 Gew.-% Mineralien umfasst.

5. Produkt nach Anspruch 1, wobei das Produkt weniger als 0,1 Gew.-% Fett, etwa 1 bis 7 Gew.-% Wasser und etwa 2 bis 20 Gew.-% Mineralien umfasst.

6. Produkt nach einem der Ansprüche 1 bis 5, wobei das Produkt Mikronähr-stoffe umfasst, die Spurenelemente und Vitamine einschließen.

7. Produkt nach einem der Ansprüche 1 bis 6, wobei die freien Aminosäuren 26 Aminosäuren umfassen: Alanin, Arginin, Asparagin, Asparaginsäure, Cystein, Glutaminsäure, Histidin, Isoleucin, Leucin, Lysin, Methionin, Phenylalanin, Prolin, Serin, Threonin, Tryptophan, Tyrosin, Valin, Cystin, Hydroxylysin, Hydroxyprolin, Ornithin, Taurin und Thyroxin.

8. Produkt nach einem der Ansprüche 1 bis 7, wobei die kurzen natürlichen Peptide eine Molekülgröße von weniger als 5 kDa aufweisen.

9. Produkt nach einem der Ansprüche 1 bis 8, wobei das Produkt Amizat ist.

10. Produkt nach einem der Ansprüche 1 bis 8, umfassend einen Gesamtgehalt an
| | |
|---|---|
| 40-70 g | Alanin pro kg des Produkts; |
| 25-60 g | Arginin pro kg des Produkts; |
| 40-85 g | Asparagin + Asparaginsäure pro kg des Produkts; |
| 0-10 g | Cystin pro kg des Produkts; |
| 80-115 g | Glutaminsäure + Glutamin pro kg des Produkts; |
| 40-70 g | Glycin pro kg des Produkts; |
| 8-30 g | Histidin pro kg des Produkts; |
| 15-45 g | Isoleucin pro kg des Produkts; |
| 35-70 g | Leucin pro kg des Produkts; |
| 35-65 g | Lysin pro kg des Produkts; |
| 10-30 g | Methionin pro kg des Produkts; |
| 15-45 g | Phenylalanin pro kg des Produkts; |
| 15-50 g | Prolin pro kg des Produkts; |
| 15-40 g | Serin pro kg des Produkts; |
| 15-45 g | Threonin pro kg des Produkts; |
| 1-15 g | Tryptophan pro kg des Produkts; |
| 10-40 g | Tyrosin pro kg des Produkts; und |
| 20-55 g | Valin pro kg des Produkts. |

11. Produkt nach einem der Ansprüche 1 bis 8, umfassend einen Gesamtgehalt an
| | |
|---|---|
| 45-60 g | Alanin pro kg des Produkts; |
| 30-45 g | Arginin pro kg des Produkts; |
| 40-70 g | Asparagin + Asparaginsäure pro kg des Produkts; |
| 1-7 g | Cystin pro kg des Produkts; |
| 80-105 g | Glutaminsäure + Glutamin pro kg des Produkts; |
| 40-70 g | Glycin pro kg des Produkts; |
| 8-25 g | Histidin pro kg des Produkts; |
| 18-40 g | Isoleucin pro kg des Produkts; |
| 35-65 g | Leucin pro kg des Produkts; |
| 40-60 g | Lysin pro kg des Produkts; |
| 12-25 g | Methionin pro kg des Produkts; |
| 18-40 g | Phenylalanin pro kg des Produkts; |
| 25-45 g | Prolin pro kg des Produkts; |
| 18-40 g | Serin pro kg des Produkts; |
| 18-40 g | Threonin pro kg des Produkts; |
| 2-15 g | Tryptophan pro kg des Produkts; |
| 15-35 g | Tyrosin pro kg des Produkts; und |
| 20-50 g | Valin pro kg des Produkts. |

12. Produkt nach einem der Ansprüche 1 bis 8, umfassend einen Gesamtge-halt an
| | |
|---|---|
| 45-57 g | Alanin pro kg des Produkts; |
| 31-44 g | Arginin pro kg des Produkts; |
| 45-68 g | Asparagin + Asparaginsäure pro kg des Produkts; |
| 1,5-7 g | Cystin pro kg des Produkts; |
| 85-100 g | Glutaminsäure und Glutamin pro kg des Produkts; |
| 45-68 g | Glycin pro kg des Produkts; |
| 10-20 g | Histidin pro kg des Produkts; |
| 20-39 g | Isoleucin pro kg des Produkts; |
| 40-64 g | Leucin pro kg des Produkts; |
| 42-58 g | Lysin pro kg des Produkts; |
| 15-22 g | Methionin pro kg des Produkts; |
| 20-38 g | Phenylalanin pro kg des Produkts; |
| 29-40 g | Prolin pro kg des Produkts; |
| 20-36 g | Serin pro kg des Produkts; |
| 22-38 g | Threonin pro kg des Produkts; |
| 4-10 g | Tryptophan pro kg des Produkts; |
| 16-30 | Tyrosin pro kg des Produkts; und |
| 26-49 g | Valin pro kg des Produkts. |

## Revendications

1. Composition de produit étant exempte de protéines comprenant une combinaison d'acides aminés et de peptides, la combinaison étant constituée d'acides aminés naturels libres et de peptides naturels courts d'une origine animale aquatique, dans laquelle
- les acides aminés libres comprennent tous les 20 acides aminés nécessaires pour la synthèse de protéines humaines ;
- les peptides naturels courts ont une taille moléculaire inférieure à 10 kDa ; et
- comprenant plus de 40 % en poids d'acides aminés libres, déterminé en tant que rapport de l'azote d'amine par titrage au formol à l'azote total par le procédé de Kjeldahl.

2. Produit selon la revendication 1, comprenant plus de 50 % en poids d'acides aminés libres.

3. Produit selon la revendication 1, comprenant plus de 57 % en poids d'acides aminés libres et moins de 43 % en poids de peptides courts et où les acides aminés et les peptides courts représentent plus de 68 % en poids du produit.

4. Produit selon la revendication 1, où ledit produit comprend moins de 0,7 % en poids de matière grasse, environ 1 à 7 % en poids d'eau et environ 2 à 20 % en poids de minéraux.

5. Produit selon la revendication 1, où ledit produit comprend moins de 0,1 % en poids de matière grasse, environ 1 à 7 % en poids d'eau et environ 2 à 20 % en poids de minéraux.

6. Produit selon l'une quelconque des revendications 1 à 5, où le produit comprend des micro-nutriments incluant des oligoéléments et des vitamines.

7. Produit selon l'une quelconque des revendications 1 à 6, où les acides aminés libres comprennent 26 acides aminés : alanine, arginine, asparagine, acide aspartique, cystéine, acide glutamique, glutamine, glycine, histidine, isoleucine, leucine, lysine, méthionine, phénylalanine, proline, sérine, thréonine, tryptophane, tyrosine, valine, cystine, hydroxylysine, hydroxyproline, ornithine, taurine et thyroxine.

8. Produit selon l'une quelconque des revendications 1 à 7, dans lequel les peptides naturels courts ont une taille moléculaire inférieure à 5 kDa.

9. Produit selon l'une quelconque des revendications 1 à 8, où le produit est l'Am izate.

10. Produit selon l'une quelconque des revendications 1 à 8, comprenant une quantité totale de
| | |
|---|---|
| 40 à 70 g | d'alanine par kg de produit ; |
| 25 à 60 g | d'arginine par kg de produit ; |
| 40 à 85 g | d'asparagine + acide aspartique par kg de produit ; |
| 0 à 10 g | de cystine par kg de produit ; |
| 80 à 115 g | d'acide glutamique + glutamine par kg de produit ; |
| 40 à 70 g | de glycine par kg de produit ; |
| 8 à 30 g | d'histidine par kg de produit ; |
| 15 à 45 g | d'isoleucine par kg de produit ; |
| 35 à 70 g | de leucine par kg de produit ; |
| 35 à 65 g | de lysine par kg de produit ; |
| 10 à 30 g | de méthionine par kg de produit ; |
| 15 à 45 g | de phénylalanine par kg de produit ; |
| 15 à 50 g | de proline par kg de produit ; |
| 15 à 40 g | de sérine par kg de produit ; |
| 15 à 45 g | de thréonine par kg de produit ; |
| 1 à 15 g | de tryptophane par kg de produit ; |
| 10 à 40 g | de tyrosine par kg de produit ; et |
| 20 à 55 g | de valine par kg de produit. |

11. Produit selon l'une quelconque des revendications 1 à 8, comprenant une quantité totale de
| | |
|---|---|
| 45 à 60 g | d'alanine par kg de produit ; |
| 30 à 45 g | d'arginine par kg de produit ; |
| 40 à 70 g | d'asparagine + acide aspartique par kg de produit ; |
| 1 à 7 g | de cystine par kg de produit ; |
| 80 à 105 g | d'acide glutamique + glutamine par kg de produit ; |
| 40 à 70 g | de glycine par kg de produit ; |
| 8 à 25 g | d'histidine par kg de produit ; |
| 18 à 40 g | d'isoleucine par kg de produit ; |
| 35 à 65 g | de leucine par kg de produit ; |
| 40 à 60 g | de lysine par kg de produit ; |
| 12 à 25 g | de méthionine par kg de produit ; |
| 18 à 40 g | de phénylalanine par kg de produit ; |
| 25 à 45 g | de proline par kg de produit ; |
| 18 à 40 g | de sérine par kg de produit ; |
| 18 à 40 g | de thréonine par kg de produit ; |
| 2 à 15 g | de tryptophane par kg de produit ; |
| 15 à 35 g | de tyrosine par kg de produit ; et |
| 20 à 50 g | de valine par kg de produit. |

12. Produit selon l'une quelconque des revendications 1 à 8, comprenant une quantité totale de
| | |
|---|---|
| 45 à 57 g | d'alanine par kg de produit ; |
| 31 à 44 g | d'arginine par kg de produit ; |
| 45 à 68 g | d'asparagine + acide aspartique par kg de produit ; |
| 1,5 à 7 g | de cystine par kg de produit ; |
| 85 à 100 g | d'acide glutamique + glutamine par kg de produit ; |
| 45 à 68 g | de glycine par kg de produit ; |
| 10 à 20 g | d'histidine par kg de produit ; |
| 20 à 39 g | d'isoleucine par kg de produit ; |
| 40 à 64 g | de leucine par kg de produit ; |
| 42 à 58 g | de lysine par kg de produit ; |
| 15 à 22 g | de méthionine par kg de produit ; |
| 20 à 38 g | de phénylalanine par kg de produit ; |
| 29 à 40 g | de proline par kg de produit ; |
| 20 à 36 g | de sérine par kg de produit ; |
| 22 à 38 g | de thréonine par kg de produit ; |
| 4 à 10 g | de tryptophane par kg de produit ; |
| 16 à 30 g | de tyrosine par kg de produit ; et |
| 26 à 49 g | de valine par kg de produit. |
